# EUROPEAN PATENT APPLICATION

(11) **EP 4 623 843 A1**
(43) Date of publication of application: **01.10.2025**
(21) Application number: 23893367.5
(22) Date of filing: 13.09.2023
(51) Int. Cl.: A61B 17/22, A61B 17/32

(54) **PLAQUE TREATMENT DEVICE**

(30) Priority: 23.11.2022 CN 202211475541
(71) Applicant: Shanghai MicroPort Medical (Group) Co., Ltd., Shanghai 201203 (CN)
(72) Inventor: SHU, Yuting, Shanghai 201203 (CN); ZHOU, Qi, Shanghai 201203 (CN)
(74) Representative: Lavoix
(86) International application number: PCT/CN2023/118460
(87) International publication number: WO 2024/109274

(57) **Abstract**

The present invention provides a plaque treatment device, which includes a catheter, a plaque disruption mechanism and a content recovery mechanism. The plaque disruption mechanism is configured to switch between a deployed configuration and a first stowed configuration. When in the first stowed configuration, the plaque disruption mechanism is loaded in or on the catheter and adapted to move therewith. When in the deployed configuration, the plaque disruption mechanism is adapted to disrupt a cap of a target plaque. The content recovery mechanism is adapted to recover the contents of the target plaque. With this arrangement, the plaque disruption mechanism can be used to mechanically disrupt the cap of the target plaque, and the contents thereof can be recovered by the content recovery mechanism, without entering the blood circulation to cause harm. This mechanical therapy is advantageous in thorough treatment of vulnerable plaque in a rapid and accurate way.

## Description

### TECHNICAL FIELD

The present invention relates to the field of medical devices and, in particular, to a plaque treatment device.

### BACKGROUND

Vulnerable plaque, also known as unstable plaque, soft plaque, high-risk plaque and so forth, is atherosclerotic plaque that develops rapidly and is prone to thrombosis, not stable and associated with a high risk. This type of plaque features a thin fibrous cap and a large lipid core, readiness to rupture, lack of stability and high likeliness of causing an acute cardiovascular or cerebrovascular event. For example, despite a low degree of stenosis (<50%), some patients with vulnerable plaque may experience an acute cardiovascular or cerebrovascular event at any time, such as myocardial infarction, cardiac arrest, acute cerebral infarction or the like. Clinical evidences show that 75% of acute myocardial infarctions are attributed to the rupture of vulnerable plaques. Therefore, vulnerable plaque is a huge threat to our life.

At present, the treatment of vulnerable plaque relies essentially on medication. For example, the most widely adopted current practice is to use statins for enhancing lipid lowering. Other medicines are also available for this purpose, such as anticoagulants and antiplatelet drugs for reducing local arterial inflammation and thereby suppressing thrombosis following plaque rupture, GP IIb/IIIa antagonists and unfractionated heparin (UFH) for enhancing perfusion and mitigating thrombosis, and calcium ion antagonists for slowing down the progression of atherosclerosis. However, all these medications are associated with a number of problems including slow effectiveness, long-term treatment and unreliable efficacy.

### SUMMARY

It is an object of the present invention to provide a plaque treatment device, which overcomes the problem that conventional vulnerable plaque treatment relies mainly on medication.

To this end, the present invention provides a plaque treatment device, which includes a catheter, a plaque disruption mechanism and a content recovery mechanism. The plaque disruption mechanism is configured to switch between a deployed configuration and a first stowed configuration.

When in the first stowed configuration, the plaque disruption mechanism is loaded in or on the catheter and adapted to move therewith. When in the deployed configuration, the plaque disruption mechanism is adapted to disrupt a cap of a target plaque. The content recovery mechanism is adapted to recover the contents of the target plaque.

Optionally, the apparatus may further include a drive mechanism for driving the plaque disruption mechanism to switch between the deployed configuration and the first stowed configuration, wherein the plaque disruption mechanism includes a sharp member coupled to the drive mechanism, and as the plaque disruption mechanism switches from the first stowed configuration to the deployed configuration, the sharp member protrudes out of the catheter radially with respect thereto to pierce the cap of the target plaque.

Optionally, the drive mechanism may include a slider and a link, the link coupled to the slider at one end and to the plaque disruption mechanism at the other end, the slider adapted to move along an axis of the catheter to drive the plaque disruption mechanism to switch between the deployed configuration and the first stowed configuration through the link.

Optionally, the drive mechanism may include a slider cavity and a drive fluid channel, both extending along the axis of the catheter, wherein a radially outer contour of the slider is complementary in shape to a radially inner contour of the slider cavity; the slider is axially movably disposed in the slider cavity; the drive fluid channel communicates with the slider cavity; and the drive fluid channel is adapted for injection or evacuation of a driving fluid into or from the slider cavity therethrough, which drives the slider to move axially.

Optionally, the drive mechanism may include a threaded drive member extending along the axis of the catheter, wherein the slider is threadedly coupled to the threaded drive member, and the threaded drive member is adapted to be rotated to drive the slider to move axially.

Optionally, the drive mechanism may include two axially spaced sliders and at least two links, each of the sliders coupled to at least one of the links, the two sliders configured to move synchronously in opposite directions.

Optionally, the plaque disruption mechanism may include an extension body disposed outside the catheter and extending along the axis thereof, wherein the sharp member is provided on the extension body, and the extension body is coupled to the other end of the link; the extension body is adapted to be driven by the link to displace radially; when the plaque disruption mechanism is in the first stowed configuration, the extension body abuts against an outer wall of the catheter; and when the plaque disruption mechanism is in the deployed configuration, the extension body is radially spaced away from the catheter and adapted to squeeze the cap of the target plaque.

Optionally, the apparatus may further include a drive mechanism for driving the plaque disruption mechanism to switch between the deployed configuration and the first stowed configuration, wherein the plaque disruption mechanism includes a sharp member coupled to the drive mechanism; the drive mechanism includes a drive wire and a guide member, the drive wire coupled to the sharp member, the guide member adapted to guide and deflect the drive wire; when the plaque disruption mechanism is in the first stowed configuration, the sharp member does not protrude out of the guide member or the catheter, whichever is more external; and as the plaque disruption mechanism switches from the first stowed configuration to the deployed configuration, the sharp member is driven by the drive wire to protrude out of the guide member and the catheter at an angle with respect to an axis of the catheter to pierce the cap of the target plaque.

Optionally, the guide member may include a curved tubular guide section, the tubular guide section including a curved segment, a proximal segment extending tangent to the curved segment along the axis of the catheter, and a distal segment extending tangent to the curved segment at the angle with respect to the axis of the catheter through an outer wall of the catheter, the tubular guide section adapted for movable passage of the drive wire therethrough and to guide and deflect the drive wire.

Optionally, the content recovery mechanism may include an occlusion member allowing selective passage of particles not larger than particles of a critical size, which are in turn not larger than some particles in the contents, the occlusion member configured to switch between an occluding configuration and a second stowed configuration, wherein:
when in the occluding configuration, the occlusion member is adapted for occlusion downstream of the target plaque and to collect particles in the contents greater than the critical size;
when in the second stowed configuration, the occlusion member is loaded in the catheter and adapted to move therewith; and
as the occlusion member switches from the occluding configuration to the second stowed configuration, the collected contents are prevented from escaping from the occlusion member.

Optionally, the plaque disruption mechanism may include a sharp member, wherein the content recovery mechanism includes a recovery bore extending through the sharp member along an axis thereof and a recovery channel extending along an axis of the catheter and communicating with the recovery bore, the recovery channel and the recovery bore adapted for evacuation of the contents of the target plaque.

Optionally, the content recovery mechanism may further include a plurality of lateral holes extending radially with respect to the sharp member through a side wall thereof and communicating with the recovery bore, the lateral holes adapted for allowing passage of the contents of the target plaque therethrough.

Optionally, the catheter may be a multi-lumen catheter.

In summary, the present invention provides a plaque treatment device, which includes a catheter, a plaque disruption mechanism and a content recovery mechanism. The plaque disruption mechanism is configured to switch between a deployed configuration and a first stowed configuration. When in the first stowed configuration, the plaque disruption mechanism is loaded in or on the catheter and adapted to move therewith. When in the deployed configuration, the plaque disruption mechanism is adapted to disrupt a cap of a target plaque. The content recovery mechanism is adapted to recover the contents of the target plaque.

With this arrangement, the plaque disruption mechanism can be used to mechanically disrupt the cap of the target plaque, and the contents thereof can be recovered by the content recovery mechanism, without entering the blood circulation to cause harm. This mechanical therapy is advantageous in thorough treatment of vulnerable plaque in a rapid and accurate way.

### BRIEF DESCRIPTION OF THE DRAWINGS

Those of ordinary skill in the art will understand that the following drawings are presented to enable a better understanding of the present invention and not intended to limit the scope thereof in any sense, in which:
Fig. 1 schematically illustrates a plaque treatment device according to an embodiment of the present invention;
Fig. 2 schematically illustrates a plaque disruption mechanism and a content recovery mechanism according to a preferred exemplary embodiment of the present invention;
Fig. 3 is an enlarged partial view of the plaque disruption mechanism of Fig. 2;
Fig. 4 shows a schematic transverse cross-sectional view of a catheter according to an embodiment of the present invention;
Fig. 5 schematically illustrates a drive mechanism according to a preferred exemplary embodiment of the present invention;
Fig. 6 schematically illustrates a drive mechanism according to another preferred exemplary embodiment of the present invention;
Fig. 7 schematically illustrates a sharp member according to a preferred exemplary embodiment of the present invention;
Fig. 8 schematically illustrates a sharp member of Fig. 3, which has penetrated into a target plaque;
Fig. 9 schematically illustrates a plaque disruption mechanism and a content recovery mechanism according to another preferred exemplary embodiment of the present invention; and
Fig. 10 schematically illustrates a sharp member of Fig. 9, which has penetrated into a target plaque.

### List of Reference Numerals

1 catheter; 2 plaque disruption mechanism; 21 sharp member; 22 extension body; 3 content recovery mechanism; 31 recovery bore; 32 recovery channel; 33 lateral hole; 34 occlusion member; 4 target plaque; 41 cap; 42 blood vessel; 5 drive mechanism; 51 slider; 52 link; 53 slider cavity; 54 drive fluid channel; 55 stopper; 56 threaded drive member; 57 guide member; 571 extension section; 572 tubular guide section.

### DETAILED DESCRIPTION

Objects, advantages and features of the present invention will become more apparent upon reading the following more detailed description of specific embodiments thereof in conjunction with the accompanying drawings. Note that the figures are provided in a very simplified form not necessarily drawn to exact scale for the only purpose of helping explain embodiments of the invention disclosed herein in a more convenient and clearer way. In addition, the illustrated structures are usually part of their real-world counterparts. In particular, as the figures tend to have distinct emphases, they are sometimes drawn to different scales.

As used herein, the singular forms "a", "an" and "the" include plural referents, and the term "or" is generally employed in the sense of "and/or", "a number of" of "at least one" and "at least two" of "two or more". Additionally, the use of the terms "first", "second" and "third" herein is intended for illustration only and is not to be construed as denoting or implying relative importance or as implicitly indicating the numerical number of the referenced items. Accordingly, defining an item with "first", "second" or "third" is an explicit or implicit indication of the presence of one or at least two such items. The terms "one end" and "other end", as well as "proximal end" and "distal end", may be used herein to generally refer to corresponding end portions including corresponding endpoints, rather than only to the endpoints. As used herein, the terms "proximal end" and "distal end" may be used with respect to a plaque treatment device, with one end being inserted inside a human body and another end extending outside the body for manipulation. In this regard, when used in relation to a component, the term "proximal end" refers to a location thereon closer to the manipulation end of the apparatus that extends outside the human body, and the term "distal end" refers to a location on the component, which is closer to the end of the apparatus that is inserted within the human body and therefore farther away from the manipulation end of the apparatus. The terms "proximal end" and "distal end" may also be used to describe a manual operation, or an operation conducted by hand. In this regard, when used in relation to a component, the term "proximal end" refers to a location thereon closer to the operator, such as a surgeon or clinician, and the term "distal end" refers to a location on the component, which is closer to the apparatus and therefore farther away from the operator. Further, as used herein, the terms "mounting", "coupling", "connecting", "disposing" and any variants thereof should be interpreted in a broad sense. When an element is referred to as being "disposed" on another element, this is generally intended to only mean that there is a connection, coupling, engagement or transmission relationship between the two elements, which may be either direct or indirect with one or more intervening elements, and should not be interpreted as indicating or implying a particular spatial position relationship between them. That is, the element may be located inside, outside, above, under, beside, or at any other location relative to the other element, unless the context clearly dictates otherwise. Those of ordinary skill in the art can understand the specific meanings of the above-mentioned terms herein, depending on their context. Furthermore, the directional terms such as "above", "below", "upper", "lower", "upward", "downward", "left", "right", and the like are used in relation to the illustrative embodiments as they are depicted in the figures, the upward or upper direction being toward the top of the corresponding figure and the downward or lower direction being toward the bottom of the corresponding figure.

The present invention seeks to overcome the problem that conventional vulnerable plaque treatment relies mainly on medication by presenting a plaque treatment device.

This invention is further described below with reference to the accompanying drawings.

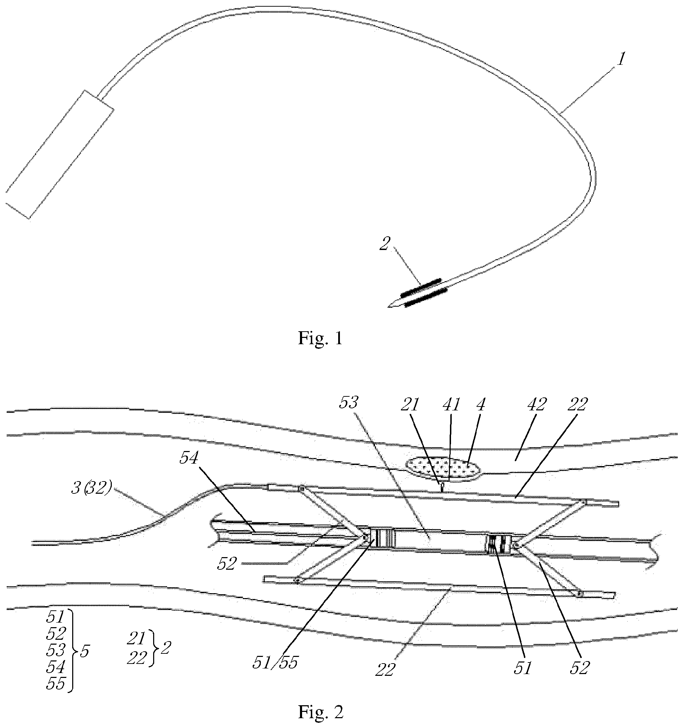

Reference is made to Figs. 1 to 8, embodiments of the present invention provide a plaque treatment device, which includes a catheter 1, a plaque disruption mechanism 2 and a content recovery mechanism 3. The plaque disruption mechanism 2 is configured to switch between a deployed configuration and a first stowed configuration. When in the first stowed configuration, the plaque disruption mechanism 2 is loaded in or on the catheter 1 and configured to move with the catheter 1. When in the deployed configuration, the plaque disruption mechanism 2 is used to disrupt a cap 41 of a target plaque 4. The content recovery mechanism 3 is adapted to recover the contents of the target plaque 41. Notably, the target plaque 4 may be, for example, a vulnerable plaque in a blood vessel of a patient, or a plaque phantom in a blood vessel model for operator training or surgical verification. The present invention is not limited to any particular type of the target plaque 4.

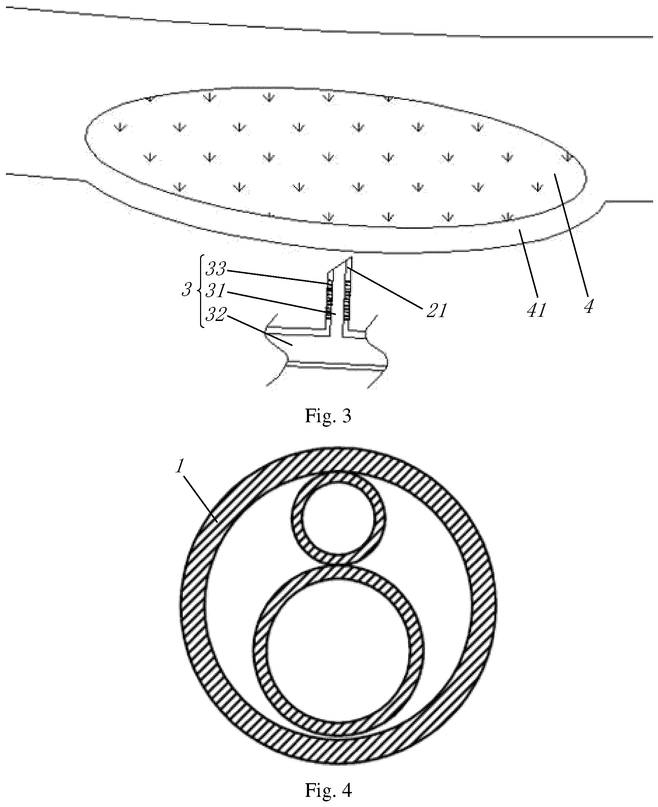

Referring to Figs. 2 and 3, the target plaque 4 may form on a side wall of a blood vessel 42, the target plaque 4 contains a substantially solid mass (e.g., a lipid core) and some liquid. The cap 41 of the target plaque 4 refers to a portion thereof facing the interior of the blood vessel 42. It will be understood that, after the cap 41 of the target plaque 4 is disrupted by the plaque disruption mechanism 2, the contents of the target plaque 4, if not treated, will leak into the blood vessel 42 and may flow with blood into a narrow lumen. This may cause an occlusion, which may lead to the occurrence of an acute cardiovascular or cerebrovascular event. To avoid this, the content recovery mechanism 3 is provided to recover the contents of the target plaque 4. With this arrangement, the plaque disruption mechanism 2 can be used to mechanically disrupt the cap 41 of the target plaque 4, and the contents thereof can be recovered by the content recovery mechanism 3, without entering the blood circulation to cause harm. This mechanical therapy is advantageous in thorough treatment of vulnerable plaque in a rapid and accurate way.

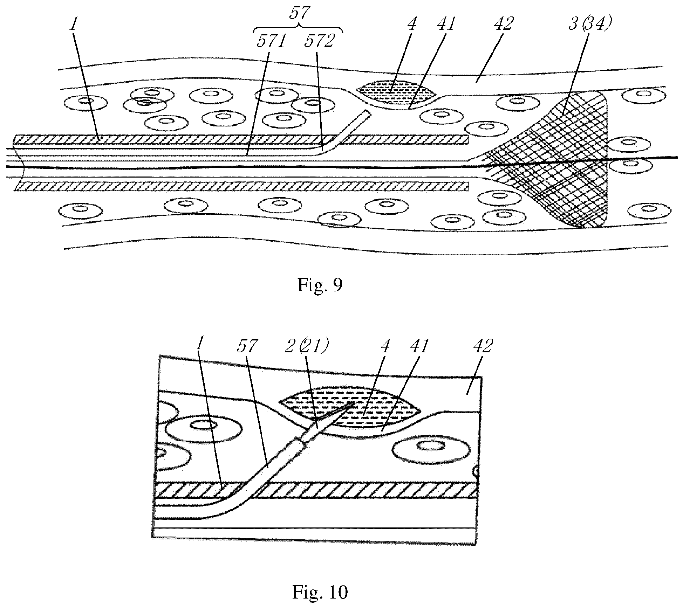

As noted above, the plaque disruption mechanism 2 is configured to switch between the deployed configuration and the first stowed configuration. In applications, the proposed apparatus is essentially intended to be introduced into the blood vessel 42 (or blood vessel model) and advanced within to the target plaque 4. In this process, the plaque disruption mechanism 2 essentially assumes the first stowed configuration, which allows it to smoothly move with the catheter 1 within the blood vessel 42 while causing no or minimal damage to a wall of the blood vessel. Notably, the plaque disruption mechanism 2 may be loaded in or on the catheter 1 in various manners so as to be in the first stowed configuration. For example, in the exemplary embodiment of Figs. 1 and 2, the plaque disruption mechanism 2 is disposed on the exterior of the catheter 1 in a radially contractible and expandable manner. In this case, the loading of the plaque disruption mechanism 2 may be accomplished by radially crimping it onto an outer circumference of the catheter 1. In some other embodiments, for example, as shown in Fig. 9, the loading of the plaque disruption mechanism 2 may be accomplished by crimping it within the catheter 1. Therefore, the present invention is not limited to any method for loading the plaque disruption mechanism 2 in or on the catheter 1, as long as it is ensured that the plaque disruption mechanism 2, when loaded in or on the catheter 1, does not adversely affect forward and backward movement of the catheter 1 within the blood vessel 42. When in the deployed configuration, the plaque disruption mechanism 2 protrudes out of the catheter 1 and can be used to pierce the cap 41. In some embodiments, the target plaque 4 may form on the side wall of a substantially straight segment of the blood vessel 42, and the plaque disruption mechanism 2 in the deployed configuration may protrude out of the catheter 1 radially with respect thereto. In some other embodiments, the target plaque 4 may form at a bend of the blood vessel 42, and the plaque disruption mechanism 2 in the deployed configuration may protrude out of the catheter 1 along an axis thereof. Those skilled in the art can understand and make modifications to this based on the general common knowledge of the art.

Preferably, the apparatus further includes a drive mechanism 5, the drive mechanism 5 is adapted to drive the plaque disruption mechanism 2 to switch between the deployed configuration and the first stowed configuration. The plaque disruption mechanism 2 includes a sharp member 21 coupled to the drive mechanism 5. After the plaque disruption mechanism 2 switches from the first stowed configuration to the deployed configuration, the sharp member 21 protrudes out of the catheter 1 radially with respect thereto and can be used to pierce the cap 41 of the target plaque 4. In some embodiments, the plaque disruption mechanism 2 may be spontaneously switchable, instead of being driven to switch, between the deployed configuration and the first stowed configuration. For example, the plaque disruption mechanism 2 may include a self-expanding mesh stent, which, when pushed out of the catheter 1 from its distal end, will spontaneously expand, thereby accomplishing the switching from the first stowed configuration to the deployed configuration. However, driving the plaque disruption mechanism 2 to switch between the deployed configuration and the first stowed configuration by the drive mechanism 5 is preferred, because this allows the plaque disruption mechanism 2 to reliably switch between the configurations with increased motion accuracy.

As shown in Fig. 2, optionally, the drive mechanism 5 may include a slider 51 and a link 52, one end of the link 52 is coupled to the slider 51, the other end of the link 52 is coupled to the plaque disruption mechanism 2. The slider 51 is adapted to move along the axis of the catheter 1 to cause the link 52 to drive the plaque disruption mechanism 2 to switch between the deployed configuration and the first stowed configuration.

In a preferred exemplary embodiment, the movement of the slider 51 may be accomplished by injecting or evacuating of a fluid (liquid or gas). Optionally, the drive mechanism 5 may include a slider cavity 53 and a drive fluid channel 54, which are arranged in line along the axis of the catheter 1. A radially outer contour of the slider 51 is complementary in shape to a radially inner contour of the slider cavity 53, and the slider 51 is axially movably disposed in the slider cavity 53. The drive fluid channel 54 communicates with the slider cavity 53, and the drive fluid channel 54 is adapted for injection of the driving fluid into the slider cavity 53, or adapted for evacuation of the driving fluid from the slider cavity 53 therethrough, thus driving the slider 51 to move axially.

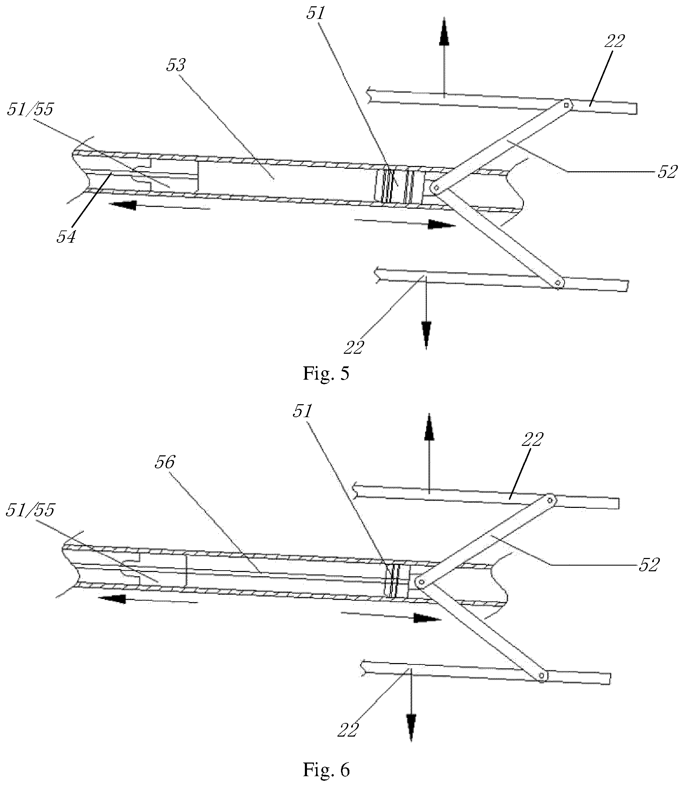

Referring to Figs. 4 and 5, optionally, the catheter 1 may be a multi-lumen catheter defining multiple lumens radially side by side, one of which forms the slider cavity 53. In one embodiment, a stopper 55 is provided at a proximal end of the slider cavity 53, and the slider 51 is movably disposed around a distal end of the slider cavity 53. Preferably, the radially outer contour of the slider 51 and the radially inner contour of the slider cavity 53 are both circular, and an outer diameter of the slider 51 matches an inner diameter of the slider cavity 53. The slider 51 is able to slide while remaining in sealing contact with the slider cavity 53, thereby closing the interior volume of the slider cavity 53. With this arrangement, when the driving fluid is injected into the slider cavity 53 through the drive fluid channel 54, the slider 51 will be pushed to move distally (towards the right hand side of Fig. 5). On the contrary, when the driving fluid is sucked away from the slider cavity 53 through the drive fluid channel 54, the slider 51 will be pushed to move proximally (towards the left hand side of Fig. 5). Optionally, the drive fluid channel 54 may also be formed by a lumen in the catheter 1. Preferably, the slider cavity 53 and the drive fluid channel 54 may be formed by the same single lumen and separated from each other by the stopper 55. Additionally, the stopper 55 defines a bore extending axially therethrough, which brings the slider cavity 53 and the drive fluid channel 54 in communication with each other. The drive fluid channel 54 in the catheter 1 may be proximally located outside the body of a patient, allowing the operator to inject or evacuate the driving fluid into or from the drive fluid channel 54 through the proximal end. This arrangement can facilitate radial shrinkage of the catheter 1, contributing to increased crossability thereof. Of course, in some other embodiments, the drive fluid channel 54 may also be provided by a different lumen in the catheter 1 from that of the slider cavity 53, or by a separate channel attached to the exterior of the catheter 1. The present invention is not limited in this regard.

Further, in one embodiment, the plaque disruption mechanism 2 includes an extension body 22 disposed outside the catheter 1, the extension body 22 extends along the axis of the catheter 1 and the sharp member 21 is provided on the extension body 22. The extension body 22 is coupled to the other end of the link 52, the extension body 22 is adapted to be driven by the link 52 to move radially. When the plaque disruption mechanism 2 is in the first stowed configuration, the extension body 22 resides on the exterior of the catheter 1. In the deployed configuration of the plaque disruption mechanism 2, the extension body 22 is radially urged away from the catheter 1 to squeeze the cap 41 of the target plaque 4. Optionally, the opposing ends of the link 52 may be hinged to the extension body 22 and the slider 51. Optionally, the end of the link 52 that is coupled to the slider 5 may be disposed within the catheter 1, while the end coupled to the extension body 22 may be located outside the catheter 1. Accordingly, the link 52 needs to be inserted through the catheter 1. To this end, slots may be formed in the catheter 1 at locations corresponding to the link 52, and the link 52 may be inserted through the slots. In this way, axial sliding of the slider 51 can be converted to radial displacement of the extension body 22, causing the sharp member 21 to move radially to pierce the cap 41.

Preferably, the plaque disruption mechanism 2 includes at least two extension bodies 22, the at least two extension bodies 22 are equidistantly spaced circumferentially around an axis of the slider cavity 53, and each of the extension bodies 22 is coupled to the slider 51 by respective links 52. With this arrangement, as a result of axial movement of the slider 51, the at least two extension bodies 22 can uniformly displace outwardly in different directions. Notably, when the plaque disruption mechanism 2 includes at least two extension bodies 22, the sharp member 21 is only provided on one of the extension bodies 22, and the remaining extension body or bodies 22 are only adapted to abut against an inner wall of the blood vessel 42 to provide backup support to the extension body 22 provided with the sharp member 21, enabling the extension bodies 22 provided with the sharp member 21 to press the target plaque 4, thereby squeezing the contents of the target plaque 4 as much as possible. In the exemplary embodiment of Fig. 5, the plaque disruption mechanism 2 includes two extension bodies 22, which are arranged in symmetry with respect to the axis of the slider cavity 53. In some other embodiments, the extension body 22 may also be provided as a cylindrical mesh coupled to the slider 51 by multiple links 52. With this arrangement, axial movement of the slider 51 can cause uniform radial expansion or contraction of the extension body 22. Optionally, the extension body or bodies 22 may be provided in the form of arms or plates, for example, and the number, dimensions, cross-sectional shapes, circumferential extents, spatial arrangement and other parameters may depend on the manufacturing circumstances and on an intended squeezed area of the target plaque 4. Preferably, the extension body or bodies 22 may be made of a biocompatible material, such as stainless steel, a titanium alloy or an organic polymer. It or they should possess a desirable degree of strength, as required for squeezing of the target plaque 4.

With continued reference to Fig. 5, in another optional embodiment, the drive mechanism 5 includes two axially-spaced sliders 51 and at least two links 52. Each of the sliders 51 is coupled to at least one of the links 52. The two sliders 51 are configured to move synchronously in opposite directions. In this case, the stopper 55 fixed at the proximal end of the slider cavity 53 is replaced with one of the sliders 51, and the interior volume of the slider cavity 53 is instead closed by the two sliders 51. With this arrangement, when the driving fluid is injected or evacuated into or from the slider cavity 53 through the drive fluid channel 54, the two sliders 51 will move synchronously in opposite directions. Further, each of the two sliders 51 is coupled by associated links 52 to the extension body or bodies 22. In this way, the synchronous movement of the two sliders 51 in opposite directions can drive two axial ends of the extension body or bodies 22 to simultaneously move radially, resulting in smooth radial displacement of the extension body or bodies 22, for example, normally to the axis of the slider cavity 53. Optionally, a proximal one of the sliders 51 defines a bore extending axially therethrough, the bore provided on the sliders 51 brings the slider cavity 53 and the drive fluid channel 54 in communication with each other.

Referring to Fig. 6, in another preferred exemplary embodiment, the movement of the slider 51 may be mechanically accomplished by threads. Optionally, the drive mechanism 5 may include a threaded drive member 56 oriented along the axis of the catheter 1. The slider 51 is threadedly coupled to the threaded drive member 56, and the threaded drive member 56 is adapted to be rotated to drive the slider 51 to move axially. In one embodiment, the threaded drive member 56 is a lead screw with an external thread, and the slider 51 has an internal thread engageable with the external thread of the lead screw. With this arrangement, the threaded drive member 56 can be rotated to cause axial movement of the slider 51. In another embodiment, the threaded drive member 56 may be a sleeve with an internal thread, while the slider 51 may define an external thread engageable with the internal thread of the sleeve. This arrangement also enables axial movement of the slider 51 as a result of the threaded drive member 56 being rotated. Likewise, the drive mechanism 5 may include two axially-spaced sliders 51. The two sliders 51 are both threadedly coupled to the threaded drive member 56. The two sliders 51 have opposite threads, and accordingly, the threaded drive member 56 also has two opposite threads engageable with the respective threads of the two sliders 51. With this arrangement, when the threaded drive member 56 is rotated, the two sliders 51 will be synchronously driven to move in sync in opposite directions.

Optionally, referring to Fig. 3, in a preferred exemplary embodiment, the content recovery mechanism 3 includes a recovery bore 31 extending through the sharp member 21 along an axis of the sharp member 21. The content recovery mechanism 3 further includes a recovery channel 32 extending along the axis of the catheter 1, the recovery bore 31 communicates with the recovery channel 32. The recovery channel 32 and the recovery bore 31 are configured for sucking out of the contents of the target plaque 4. In this preferred exemplary embodiment, the sharp member 21 is generally a hollow needle pointed at one end, which can penetrate through the cap 41 into the target plaque 4. Preferably, the extension body 22 carrying the sharp member 21 defines a hollow cavity, which is distally closed and proximally brought into communication with one lumen of the catheter 1 by a coupling tube, and the recovery bore 31 communicates with the cavity of the extension body 22. Thus, the cavity of the extension body 22, the coupling tube and the lumen of the catheter 1 are communicatively connected sequentially from distal to proximal and together constitute the recovery channel 32. The proximal end of the recovery channel 32 is located outside a patient's body, allowing an operator to recover, e.g., by suction, the contents of the target plaque 4 through the recovery channel 32 and the recovery bore 31, or to squeeze the contents out of the target plaque 4 through the extension body or bodies 22. It will be understood that, in order to achieve smooth removal of the contents, inner diameters of the recovery channel 32 and the recovery bore 31 should be sized larger than the contents.

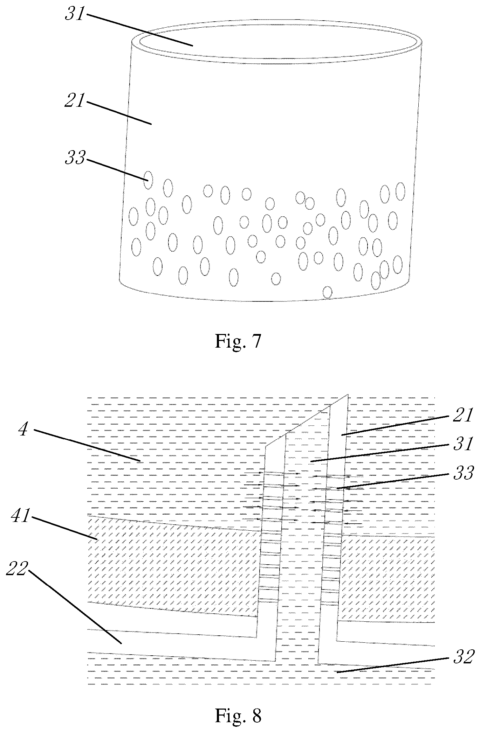

Referring to Figs. 7 and 8, the content recovery mechanism 3 further has multiple lateral holes 33, the lateral holes 33 extend through a side wall of the sharp member 21 along the radial direction of the sharp member 21 and communicate with the recovery bore 31. The lateral holes 33 allow passage of the contents of the target plaque 4 therethrough. With additional reference to Fig. 3, the recovery of the contents of the target plaque 4 by the content recovery mechanism 3 starts with the sharp member 21 penetrating through the cap 41 into the target plaque 4. In this initial stage, the contents of the target plaque 4 can enter the recovery channel 32 through the recovery bore 31 of the sharp member 21. Subsequently, the extension body 22 is displaced outwardly and brought into abutment against the cap 41 to squeeze the target plaque 4. In this stage, the remaining contents of the target plaque 4 can still enter the recovery channel 32 through the recovery bore 31 of the sharp member 21. In order to remove the contents of the target plaque 4 as thoroughly as possible, the extension body or bodies 22 may be further displaced outwardly to squeeze the target plaque 4. Consequently, the pointed tip of the sharp member 21 may penetrate through the target plaque 4 into the wall of the blood vessel, or even through the wall of the blood vessel into surrounding tissue. When this happens, as the distal end of the recovery bore 31 is not positioned within the target plaque 4, the remaining contents thereof cannot enter the recovery bore 31 anymore. That is, in this stage, as the target plaque 4 is being increasingly squeezed by the extension body or bodies 22, the remaining contents of the target plaque 4 can enter the recovery bore 31 through the lateral holes 33. It will be understood that inner diameters of the lateral holes 33 should also be sized larger than the contents in order to allow passage of the contents therethrough.

Furthermore, in order to avoid excessive damage to the blood vessel 42, the outer diameter of the sharp member 21 should be as small as possible. For example, the inner diameter of the recovery bore 31 may be slightly larger than a maximum possible size of the contents. In order to speed up the removal of the contents, the plaque disruption mechanism 2 may include two or more sharp members 21. The greater the number of sharp members 21 is, the smaller the adverse influence of the size limitations of sharp members 21 on the removal efficiency will be. In one embodiment, two or more sharp members 21 may be arranged on a single extension body 22.

Referring to Figs. 9 and 10, in another preferred exemplary embodiment, the drive mechanism 5 includes a drive wire (not shown) and a guide member 57. The drive wire is coupled to the sharp member 21, and the guide member 57 is adapted to guide and deflect the drive wire to drive the plaque disruption mechanism 2 to switch between the deployed configuration and the first stowed configuration. When the plaque disruption mechanism 2 is in the first stowed configuration, the sharp member 21 does not protrude out of the guide member 57 or the catheter 1, whichever is more external. As the plaque disruption mechanism 2 switches from the first stowed configuration to the deployed configuration, the sharp member 21 is driven by the drive wire to protrude out of the guide member 57 and the catheter 1 at an angle with respect to the axis of the catheter 1 to pierce the cap 41 of the target plaque 4. Specifically, in some embodiments, the catheter 1 is more external than the guide member 57, and the guide member 57 may be entirely received in the catheter 1. In these cases, when the plaque disruption mechanism 2 is in the first stowed configuration, the sharp member 21 does not protrude out of the catheter 1. Even when the sharp member 21 partially protrudes out of the guide member 57, as long as it does not protrude out of an outer wall of the catheter 1, it can be concealed and protected in the catheter 1. In some other embodiments, the guide member 57 may partially protrude out of the catheter 1, that is, the guide member 57 is more external than the catheter 1. In this case, even if the sharp member 21 protrudes out of the outer wall of the catheter 1, as long as it does not protrude out the guide member 57.

As shown in Fig. 9, in an exemplary embodiment, the guide member 57 is tubular and includes an extension section 571 in the catheter 1. For example, the extension section 571 may be provided by a lumen of the catheter 1. The guide member 57 also includes a curved tubular guide section 572. The tubular guide section 572 has a curved segment and a proximal segment, which extends tangent to the curved segment along the axis of the catheter 1 and is, for example, joined to the extension section 571. The tubular guide section 572 also has a distal segment, which extends tangent to the curved segment at an angle with respect to the axis of the catheter 1 through the outer wall of the catheter 1. The tubular guide section 572 is adapted for movable passage of the drive wire therethrough and to guide and deflect the drive wire. Optionally, the proximal end of the drive wire is passed through the extension section 571 and located outside a patient's body to enable an operator to advance or retract the drive wire. It will be understood that the drive wire is movably inserted through the guide member 57 so that its forward and backward movement is restricted and guided by the guide member 57. The sharp member 21 is disposed at a distal end of the drive wire. As the drive wire moves with the sharp member 21 at the distal end thereof, it will be increasingly deflected in the tubular guide section 572. As the drive wire and the sharp member 21 advance out of the tubular guide section 572, and hence of the catheter 1, they are oriented at said angle with respect to the axis of the catheter 1. At this point, when the drive wire is further pushed distally, the sharp member 21 will protrude out of the tubular guide section 572, thus switching the plaque disruption mechanism 2 to the deployed configuration. It will be understood that the plaque disruption mechanism 2 can be considered to remain in the first stowed configuration as long as the sharp member 21 does not protrude out of the tubular guide section 572.

The content recovery mechanism 3 described above in connection with the foregoing exemplary embodiments can be suitably used with the plaque disruption mechanism 2 of the exemplary embodiment of Figs. 9 and 10. That is, it includes the recovery bore 31 and the recovery channel 32, and after the sharp member 21 penetrates through the cap 41 into the target plaque 4, the contents of the target plaque 4 can be removed by applying suction to the proximal end of the recovery channel 32.

The present invention also provides another preferred exemplary embodiment of the content recovery mechanism 3, the content recovery mechanism 3 further includes an occlusion member 34, the occlusion member 34 allows selective passage of particles not larger than particles of a critical size, which are in turn not larger than some particles in the contents. The occlusion member 34 is configured to switch between an occluding configuration and a second stowed configuration. When in the occluding configuration, the occlusion member 34 is adapted for occlusion downstream of the target plaque 4 and to collect particles in the contents greater than the critical size. When in the second stowed configuration, the occlusion member 34 is loaded in the catheter 1 and adapted to move with the catheter 1. As the occlusion member 34 switches from the occluding configuration to the second stowed configuration, the collected contents are prevented from escaping from the occlusion member 34.

Optionally, the occlusion member 34 may include an expandable and contractible elastic structure, such as a braided mesh support made from nickel-titanium alloy wires. As such, the occlusion member 34 may be contracted and received in the catheter 1 and delivered therein into the blood vessel 42. When reaching the vicinity of the target plaque 4, the occlusion member 34 may be distally pushed out of the catheter 1. After that, the occlusion member 34 can expand to accomplish occlusion downstream of the target plaque 4. Notably, the term "downstream" is used herein with respect to a direction of blood flow through the blood vessel 42. For example, in the exemplary embodiment of Fig. 9, as blood flows from the left to the right, the occlusion member 34 is deployed on the right-hand side of the target plaque 4. Due to relatively large openings in the elastic structure of the occlusion member 34 (e.g., a mesh support), the occlusion member 34 may further include a semipermeable membrane covering the elastic structure. Preferably, after the occlusion member 34 expands and accomplishes occlusion downstream of the target plaque 4, the semipermeable membrane and the elastic structure span the entire transverse cross-section of the blood vessel 42. The semipermeable membrane allows selective permeation of particles not larger than some particles in the contents, as well as of blood cells and other blood components. In this way, after the occlusion member 34 expands and accomplishes occlusion downstream of the target plaque 4, large particles contained in the target plaque 4, which may cause thrombosis, can be retained on the blood inflow side of the semipermeable membrane, without entering the blood circulation to cause some risks.

Additionally, as the occlusion member 34 switches from the occluding configuration to the second stowed configuration, the collected contents are prevented from escaping from the occlusion member 34. In one exemplary embodiment, the elastic structure of the occlusion member 34 may be, for example, shaped like an umbrella and proximally moved into the catheter 1 without escape of the contents collected therein.

Preferably, in the exemplary embodiment of the Figs. 9 and 10, the sharp member 21 may be shaped like a blade. With this arrangement, it can both pierce and cut open the cap 41 of the target plaque 4, allowing rapid release of the contents of the target plaque 4 into the blood vessel 42.

During use, the catheter 1 may be delivered on a guidewire to the vicinity of the target plaque 4, and then the catheter 1 circumferentially rotates so that the distal segment of the tubular guide section 572 is oriented in alignment with the target plaque 4. At this time, the occlusion member 34 may be released downstream of the target plaque 4 and switch to the occluding configuration to occlude the blood vessel 42. The drive wire and the sharp member 21 may be then advanced out of the catheter 1 under the guidance of the tubular guide section 572 so that the sharp member 21 is aligned with the target plaque 4. At this point, the sharp member 21 may be manipulated to pierce the cap 41 of the target plaque 4, resulting in release of the contents of the target plaque 4. The contents will flow in blood to the vicinity of the occlusion member 34 and then blocked and collected on the semipermeable membrane of the occlusion member 34 under the action of blood pressure. After the contents of the target plaque 4 are completely filtered out and retained on the semipermeable membrane of the occlusion member 34, the occlusion member 34 may be withdrawn. Specifically, it may cause the occlusion member 34 to switch to the second stowed configuration, the occlusion member 34 is loaded in the catheter 1 together with the filtered and collected contents, and then withdrawn from the patient's body.

It will be understood that the content recovery mechanism 3 including the occlusion member 34 is not limited to applying to only the preferred exemplary embodiment of Figs. 9 and 10, but may also apply to the preferred exemplary embodiment of Fig. 2. In addition, the content recovery mechanism 3 is not limited to including either of the occlusion member 34 and the recovery bore 31, but may include both. For example, the exemplary embodiment of Fig. 2 may be combined with the occlusion member 34. In this way, even when a small fraction of the contents leaks from the cap 41 during squeezing, it can be recovered by the occlusion member 34, resulting in higher reliability and safety.

In summary, the present invention provides a plaque treatment device, which includes a catheter, a plaque disruption mechanism and a content recovery mechanism. The plaque disruption mechanism is configured to switch between a deployed configuration and a first stowed configuration. When in the first stowed configuration, the plaque disruption mechanism is loaded in or on the catheter and adapted to move therewith. When in the deployed configuration, the plaque disruption mechanism is adapted to disrupt a cap of a target plaque. The content recovery mechanism is adapted to recover the contents of the target plaque. With this arrangement, the plaque disruption mechanism can be used to mechanically disrupt the cap of the target plaque, and the contents thereof can be recovered by the content recovery mechanism, without entering the blood circulation to cause harm. This mechanical therapy is advantageous in thorough treatment of vulnerable plaque in a rapid and accurate way.

It should be noted that the foregoing embodiments may be combined in any suitable combination. The description presented above is merely that of a few preferred embodiments of the present invention and is not intended to limit the scope thereof in any sense. Any and all changes and modifications made by those of ordinary skill in the art based on the above teachings fall within the scope of the invention.

## Claims

1. A plaque treatment device, comprising a catheter, a plaque disruption mechanism and a content recovery mechanism, the plaque disruption mechanism configured to switch between a deployed configuration and a first stowed configuration,
wherein when in the first stowed configuration, the plaque disruption mechanism is loaded in or on the catheter and adapted to move with the catheter; when in the deployed configuration, the plaque disruption mechanism is adapted to disrupt a cap of a target plaque; and the content recovery mechanism is adapted to recover contents of the target plaque.

2. The plaque treatment device according to claim 1, further comprising a drive mechanism for driving the plaque disruption mechanism to switch between the deployed configuration and the first stowed configuration, wherein the plaque disruption mechanism comprises a sharp member coupled to the drive mechanism, and as the plaque disruption mechanism switches from the first stowed configuration to the deployed configuration, the sharp member protrudes out of the catheter radially with respect to the catheter to pierce the cap of the target plaque.

3. The plaque treatment device according to claim 2, wherein the drive mechanism comprises a slider and a link, one end of the link coupled to the slider and the other end of the link coupled to the plaque disruption mechanism, the slider adapted to move along an axis of the catheter to drive the plaque disruption mechanism to switch between the deployed configuration and the first stowed configuration through the link.

4. The plaque treatment device according to claim 3, wherein the drive mechanism comprises a slider cavity and a drive fluid channel, both extending along the axis of the catheter, wherein a radially outer contour of the slider is complementary in shape to a radially inner contour of the slider cavity; the slider is axially movably disposed in the slider cavity; the drive fluid channel communicates with the slider cavity; and the drive fluid channel is adapted for injection of a driving fluid into the slider cavity or evacuation of a driving fluid from the slider cavity therethrough, thereby driving the slider to move axially.

5. The plaque treatment device according to claim 3, wherein the drive mechanism comprises a threaded drive member extending along the axis of the catheter, wherein the slider is threadedly coupled to the threaded drive member, and the threaded drive member is adapted to be rotated to drive the slider to move axially.

6. The plaque treatment device according to any one of claims 3 to 5, wherein the drive mechanism comprises two axially spaced sliders and at least two links, each of the sliders coupled to at least one of the links, the two sliders configured to move synchronously in opposite directions.

7. The plaque treatment device according to claim 3, wherein the plaque disruption mechanism comprises an extension body disposed outside the catheter, and the extension body extending along the axis of the catheter, wherein the sharp member is provided on the extension body, and the extension body is coupled to the other end of the link; the extension body is adapted to be driven by the link to displace radially; when the plaque disruption mechanism is in the first stowed configuration, the extension body abuts against an outer wall of the catheter; and when the plaque disruption mechanism is in the deployed configuration, the extension body is radially spaced away from the catheter and adapted to squeeze the cap of the target plaque.

8. The plaque treatment device according to claim 1, further comprising a drive mechanism for driving the plaque disruption mechanism to switch between the deployed configuration and the first stowed configuration, wherein the plaque disruption mechanism comprises a sharp member coupled to the drive mechanism; the drive mechanism comprises a drive wire and a guide member, the drive wire coupled to the sharp member, the guide member adapted to guide and deflect the drive wire; when the plaque disruption mechanism is in the first stowed configuration, the sharp member does not protrude out of the guide member or the catheter, whichever is more external; and as the plaque disruption mechanism switches from the first stowed configuration to the deployed configuration, the sharp member is driven by the drive wire to protrude out of the guide member and the catheter at an angle with respect to an axis of the catheter to pierce the cap of the target plaque.

9. The plaque treatment device according to claim 8, wherein the guide member comprises a curved tubular guide section, the tubular guide section comprising a curved segment, a proximal segment extending tangent to the curved segment along the axis of the catheter, and a distal segment extending tangent to the curved segment at the angle with respect to the axis of the catheter through an outer wall of the catheter, the tubular guide section adapted for movable passage of the drive wire therethrough and to guide and deflect the drive wire.

10. The plaque treatment device according to claim 1, wherein the content recovery mechanism comprises an occlusion member allowing selective permeation of particles with a size not larger than that of some particles in the contents, the occlusion member configured to switch between an occluding configuration and a second stowed configuration, wherein:
when in the occluding configuration, the occlusion member is adapted for occlusion downstream of the target plaque and to collect particles in the contents with a size greater than the size of the selective permeation of particles;
when in the second stowed configuration, the occlusion member is loaded in the catheter and adapted to move with the catheter; and
as the occlusion member switches from the occluding configuration to the second stowed configuration, the collected contents are prevented from escaping from the occlusion member.

11. The plaque treatment device according to claim 1, wherein the plaque disruption mechanism comprises a sharp member, wherein the content recovery mechanism comprises a recovery bore extending through the sharp member along an axis of the sharp member and a recovery channel extending along an axis of the catheter, the recovery bore communicating with the recovery channel, and the recovery channel and the recovery bore adapted for evacuation of the contents of the target plaque.

12. The plaque treatment device according to claim 11, wherein the content recovery mechanism further comprises a plurality of lateral holes extending radially with respect to the sharp member through a side wall of the sharp member, the plurality of lateral holes communicating with the recovery bore, and the plurality of lateral holes adapted for allowing passage of the contents of the target plaque therethrough.

13. The plaque treatment device according to claim 1, wherein the catheter is a multi-lumen catheter.
